# EUROPÄISCHE PATENTANMELDUNG

(11) **EP 2 201 916 A2**
(43) Veröffentlichungstag der Anmeldung: **30.06.2010**
(21) Anmeldenummer: 10003174.9
(22) Anmeldetag: 30.11.2000
(51) Int. Cl.: A61F 5/445

(54) **Absorptionskörper zum Anschluss an Haut- und Schleimhautoberflächen**

(30) Priorität: 19.01.2000 DE 20000887 U
(62) Teilanmeldung aus: 00977599.0
(71) Anmelder: Riesinger, Birgit, 48346 Ostbevern (DE)
(72) Erfinder: Riesinger, Birgit, 48346 Ostbevern (DE)
(74) Vertreter: Michalski Hüttermann & Partner Patentanwälte

(57) **Zusammenfassung**

Die Erfindung betrifft einen Absorptionskörper (100, 200, .....) zum Anschluss an Haut- und Schleimhautoberflächen, insbesondere zum Aufsaugen von flüssigen Ausscheidungen aus natürlichen und nicht-natürlichen, sowie aus erkrankungsbedingten menschlichen Körperöffnungen und -höhlen oder aus chirurgisch angelegten Organausleitungen, enthaltend eine Menge an Absorptionsmaterial (1), wie aufsaugendes Vlies oder ein Gemisch aus mehreren Absorptionssubstanzen, das mit wenigstens einer flüssigkeitsdurchlässigen Abdeckschicht hinterlegt ist. Der Absorptionskörper gemäß Erfindung ist **dadurch gekennzeichnet, dass** das Absorptionsmaterial (1) von einer Hülle (2) umgeben ist, die aus einem uni- oder bidirektional flüssigkeitsdurchlässigen, schleimhautverträglichen bzw. hautverträglichen Natur- oder Kunststoff, wie perforierter Vlies oder maschiges Textilmaterial, besteht, an dem die Ausscheidungen nicht oder kaum haften und das die Flüssigkeiten durch sich durchtreten lässt und eine Barriere für feste grobe Ausscheidungen bildet und die Aufnahme bzw. das Aufsaugen von entfernungspflichtigen Substanzen durch das innerhalb der Hülle (2) angeordnete Absorptionsmaterial (1) ermöglicht, so dass der direkte Kontakt der Ausscheidungen mit der Haut bzw. Schleimhaut im angeschlossenen Zustand des Absorptionskörpers an die Körperöffnung oder -höhle eingeschränkt ist, und die ausfließenden Ausscheidungen durch die Hülle (2) hindurchtreten, in das Absorptionsmaterial (1) gelangen und innerhalb der Hülle (2) räumlich fixiert verbleiben. Das Material der Hülle (2) ist so strukturiert, dass die aufzusaugenden Ausscheidungen einfacher durch die Hülle (2) durchdringen können und die innerhalb der Hülle (2) verbleibenden, flüssigen Moleküle einen erschwerten Rücklauf haben.

## Beschreibung

Die Erfindung betrifft einen Absorptionskörper zum Anschluß an Haut- und Schleimhattoberflächen, insbesondere zum Aufsaugen von flüssigen Ausscheidungen aus natürlichen und nicht-natürlichen, sowie aus erkrankungsbedingten menschlichen Körperöffungen und -höhlen oder aus chirurgisch angelegten Organausleitungen, enthaltend eine Menge an Absorptionsmaterial, wie aufsaugendes Vlies oder ein Gemisch aus mehreren Absorptionssubstanzen, das mit wenigstens einer flüssigkeitsdurchlässigen Abdeckschicht hinterlegt ist.

In DE-AS 19 62 331 ist ein hochsaugfähiger, faseriger Absorptionskörper beschrieben, mit dem kollagenhaltige, textile Außenschichten fest verbunden sind. Die textile Außenschicht ist aus einem Fasergemisch aus Zellwolle und Zellulose, oder aus Synthesefasern hergestellt. Das entstandene Flachgebilde kann als Wundauflagematerial bzw. als Wundkissen verwendet werden, das direkt auf die Wunde aufzubringen ist. Nachteilig bei dem bekannten Absorptionskörper ist, daß er nur in der ersten Wundheilungsphase wirksam ist. Bei beginnender Epithelisation werden die Teilchen des neu gebildeten Hautgewebes mitgerissen, sobald das Wundkissen von der Wunde entfernt wird. Dies betrifft auch die aus Synthesefasern bestehende Abdeckung, die eine verbesserte Durchlässigkeit der Ausscheidungen gegenüber den Naturfasern aufweist. Das Wundkissen ist relativ teuer in Herstellung wegen Verfestigung und Verschweißung des Wundauflagematerials. Die Verwendung des beschriebenen Wundkissens ist zum Aufsaugen von flüssigen Ausscheidungen aus Wunden beschränkt.

Aufgabe der Erfindung ist, einen technisch vereinfachten und kostengünstigen Absorptionskörper zu konzipieren, der sich durch verbesserte Saugfähigkeit auszeichnet. Eine weitere Aufgabe der Erfindung ist, die Einsatzbereiche des Absorptionskörpers zu erweitern, insbesondere auf chirurgisch angelegte Organausleitungen und auf natürliche und nichtnatürliche menschliche Körperöffungen und -höhlen, wie künstliche darma- und/oder fistelartige Ausgänge.

Diese Aufgabe ist durch einen Absorptionskörper der eingangs genannten Art gelöst, der dadurch gekennzeichnet ist, daß
- das Absorptionsmaterial von einer Hülle umgeben ist, die aus einem uni- oder bidirektional flüssigkeitsdurchlässigen, schleimhautverträglichen bzw. hautvertäglichen Natur- oder Kunststoff, wie perforierter Vlies oder maschiges Textilmaterial, besteht, an dem die Ausscheidungen nicht oder kaum haften und das die Flüssigkeiten durch sich durchtreten läßt und eine Barriere für feste grobe Ausscheidungen bildet und die Aufnahme bzw. das Aufsaugen von entfernungspflichtigen Substanzen durch das innerhalb der Hülle angeordnete Absorptionsmaterial ermöglicht,
- so daß der direkte Kontakt der Ausscheidungen mit der Haut bzw. Schleimhaut im angeschlossenen Zustand des Absorptionskörpers an die Körperöffung oder -höhle eingeschränkt ist, und die ausfließenden Ausscheidungen durch die Hülle hindurchtreten, in das Absorptionsmaterial gelangen und innerhalb der Hülle räumlich fixiert verbleiben.

Gemäß Unteranspruch 2 kann das Material der Hülle derart strukturiert sein, daß seine die Maschen begrenzenden Fäden- bzw. Faserabschnitte - im Schnitt durch die Hülle gesehen - jeweils etwa bogenförmig sind und mit ihren Bogen-Scheiteln nach außen gerichtet sind. Diese Struktur hat sich als besonders vorteilhaft erwiesen, da zum ersten die aufzusaugenden Ausscheidungen schneller durch die Perforation bzw. durch die Maschen durchdringen können und zum zweiten die aktive Kontaktfläche der Hülle mit der Wunde gegenüber einer herkömmlichen, flachen Materialstruktur wesentlich verkleinert ist. Im günstigsten Fall kommen mit der Oberfläche der Wunde nur die Bogen-Scheitel in Kontakt. Dadurch läßt sich die Hülle auch einfacher von der Wunde entfernen. Zum dritten wird die vorgenannte räumliche Fixierung der Ausscheidungen innerhalb der Hülle dadurch unterstützt, daß die flüssigen Moleküle einen erschwerten Rücklauf haben, falls noch nicht gänzlich durch das Absorptionsmaterial aufgesaugt worden sind.

Zur Verbesserung der Saugfähigkeit des Absorptionskörpers trägt auch die Maschengröße der Hülle 2 bei. Die Maschengröße kann im Bereich 0,1 mm bis 10,0 mm, vorzugsweise 0,25 mm bis 1,2 mm liegen. Eine unterhalb dieses Bereichs liegende Maschengröße wäre in einigen Fällen für den Transport der Ausscheidungen, insbesondere dickflüssigen, ungünstig.

Als Material der Hülle eignen sich insbesondere perforierte bzw. maschenartige Polyolefin-Folien, wie Polyethylen- oder Polypropylen-Folie, oder ein Naturstoff-Gewebe oder -vlies, das mit hautverträglichen Substanzen, wie Silicon, Paraffin, Wachs und dgl. imprägniert ist.

Der im wesentlichen flächenhafte Absorptionskörper kann oval, kreisrund, polygonal bzw. trapez- oder tropfenförmig sein. Es ist nicht ausgeschlossen, den Absorptionskörper in einer deutlich dreidimensionalen Form, wie Walze oder Kugel, herzustellen.

Diese Struktur des Materials mit bogenförmigen Fäden- oder Faserabschnitten kann auch bei einer abweichenden Auffangbeutel-Konstruktion ausgenutzt sein, bei der wenigstens eine aus dem Material bestehende Trennwand innerhalb des Auffangbeutels angeordnet ist.

Das innerhalb der Hülle angeordnete Absorptionsmaterial kann aus einem Kunststoff-Gewebe oder -vlies, beispielsweise aus Zellstoffasern gebildet sein. Es ist auch vorgesehen, das Absorptionsmaterial mit einem an sich bekannten, sogenannten Superabsorber, beispielsweise mit einem Natriumacrylat-Acrylsäure-Polymerisat zu durchsetzen.

Das Absorptionsmaterial kann auch gel- oder schwammartig sein. Es ist auch denkbar, als Absorptionsmaterial ein flächenhaftes, schwammartiges Gebilde zu verwenden, das offenporig und mit einem Hydrogel durchsetzt ist. Das Absorptionsmaterial, darin Hydrogel kann mit wundheilungfördernden Materialien angereichert sein, beispielsweise mit lytischen Enzymen aus der Gruppe von Proteasen oder mit Peptiden und/oder mit Antibiotika. Hierbei empfiehlt sich, zur Herstellung des Hydrogels ein Heteropolysaccharid, wie Agar-Agar, oder andere bioverträgliche Substanzen zu verwenden.

Schließlich ist möglich, anstelle eines flächenhaften Textilmaterials in der Hülle eine die Flüssigkeiten absorbierende Schüttung unterzubringen. Das in Schüttungsform vorliegende Absorptionsmaterial kann aus der Gruppe Natriumacrylat-Acrylsäure-Polymerisate, Polyacrylate auf Acryl- oder Methylacrylsäurebasis, Polyvinylalkohol-Maleinsäureanhydrid-Copolymere, Polysaccharid-Maleinsäureanhydrid-Copolymere, Maleinsäurederivate, Acrylamidopropansulfonsäure-Copolymere, Stärke- Acrylnitril-Pfropfpolymere, gelantinisierte Stärkederivate, Alkyl- oder Hydroxyalkylcellulose, Carboxymethylcellulose, Stärke-Acrylsäure-Pfropfpolymere, Vinylacetat-Acrylsäureester-Copolymere, Acrylnitril- oder Acrylamid-Copolymere gewählt sein.

Sowohl die Hülle, als auch das innerhalb der Hülle liegende Absorptionsmaterial kann mit einem geruchshemmenden und/oder -neutralisierenden bzw. -maskierenden Zusatz versehen sein, beispielweise mit Aktivkohlefilter oder mit Zusätzen, mit denen aktiv z.B. Schwefel-Wasserstoff-Verbindungen aus dem gasförmigen Milieu entfernt werden können. Als geruchshemmende können Naturstoffe gelten, bzw. Extrakte daraus, bioinerte Materialien sowie Kunststoffe, die eine bestimmte Elektronegativität aufweisen, wodurch eine Anlagerung und Bindung von Schwefel-Wasserstoff-Verbindungen erfolgen kann. Hierbei weisen die Kunststoffmoleküle bestimmte geometrische Formen auf, wie Prismen oder Spiralen.

Insgesamt ist das so ausgelegte Absorptionsmaterial, sowie die oben beschriebene Hülle in der Lage, ein inneres, wundheilungsfördendes Feuchtklima zu erzeugen.

Zwischen dem Absorptionsmaterial und der äußeren Hülle kann eine zusätzliche, innere Umhüllung aus einem Material, wie Baumwolle oder Zellstoff, angeordnet sein, dessen Saugfähigkeit gering bzw. kleiner als die des eigentlichen Absorptionsmaterials ist. Eine solche innere Umhüllung erschwert den direkten Kontakt zwischen den Schleimhautzellen an der Wunde oder Körperhöhle und dem Absorptionsmaterial. Anstelle der inneren Umhüllung kann zu demselben Zweck zwischen dem Absorptionsmaterial und der äußeren Hülle eine Zwischenlage in Form eines Materialabschnittes aus Baumwolle, Zellstoff oder aus Kunstfasern angeordnet sein. Der gesamte Absorptionskörper kann in einen auswechselbaren, grobmaschigen Netzbeutel eingelegt sein.

Das Absorptionsmaterial kann aus zwei oder mehr innerhalb der Hülle angeordneten, flach liegenden Lagen bestehen, die gleiche oder unterschiedliche Saugkraft aufweisen. Weiterhin kann zwischen den Lagen eine Innenwand angeordnet sein, die die Lagen voneinander vollständig oder teilweise trennt.

Der oben beschriebene Absorptionskörper kann werkseitig in einen aus Natur- oder Kunststoff hergestellten Auffangbeutel integriert sein. Vorzugsweise ist der Auffangbeutel so konstruiert, daß der in diesem untergebrachte Absorptionskörper nach dem Gebrauch entnommen werden kann. Im weitesten Sinne ist der Auffangbeutel als dichtes, vorzugsweise flächenhaftes und wiederverschließbares, elastisches Behältnis gedacht, das sowohl in der Stoma-Versorgung als auch im Inkontinenzbereich Verwendung finden kann. Darüber hinaus kann das Behältnis ein wasserdichter, elastischer Überzug sein, der mit Haltemitteln, wie Gurte, Klebestreifen und dgl., am Körper des Patienten zu befestigen ist. Der Überzug soll mit einer Öffnung zur Entnahme oder zum Auswechseln des Absorptionskörpers versehen sein. Das Behältnis kann allerdings auch in einer steifen, bzw. teilweise steifen Form vorliegen.

Der in der Stomaversorgung der Patienten zu verwendende Auffangbeutel kann in seinem Inneren einen Befestigungsbereich aufweisen, an dem der Absorptionskörper anzukleben ist. Der Befestigungsbereich kann punkt-, knopf- oder streifenförmig sein. Der Befestigungsbereich kann auch in einer unregelmäßigen Form, wie Kleks, vorliegen. Eine Klebeschicht kann auch auf den Absorptionskörper aufgebracht sein.

Eine solche Befestigung hat sich als sehr vorteilhaft erwiesen. Ohne Befestigung kann der ganze, elastische Absorptionskörper durch die festen Ausscheidungen erdrückt und somit die Saugfähigkeit des Absorptionsmaterials mechanisch vermindert werden. Hierzu kann noch eine abziehbare Schutzfolie vorgesehen sein, mit der die Klebeschicht bedeckt ist. Die Schutzfolie kann beispielsweise in Form eines zusammengefalteten Streifens, eventuell mit einer über den Klebebereich hinausragenden Lasche an der Klebeschicht abziehbar angebracht sein. Vorzugsweise ist der Absorptionskörper an einer körperentfernten Seite der Auffangbeutel-Innenwandung anzubringen.

Es können auch andere Befestigungsmechanismen Verwendung finden, mit denen der Absorptionskörper innerhalb des Auffangbeutels fixiert werden kann, beispielsweise mit Klammern oder Magnetverschlüssen.

Vorteilhaft ist, den Absorptionskörper mit einem faden- oder bandförmigen Zugmittel zu versehen, mit dem sich der Absorptionskörpers aus dem Auffangbeutel einfacher herausziehen läßt.

Der mit dem Absorptionskörper ausgestattete Überzug kann seine Dienste insbesondere gut bei Inkontinenzsystemen erweisen. Beispielsweise kann er an einen an sich bekannten Blasenkatheter angeschlossen werden. Dem Überzug kann auch ein weiterer Überzug oder ein wasserdichter Urinbeutel nachgeschaltet sein.

Weiterhin ist vorgesehen, die Hülle und/oder den Überzug an ihrem/seinem Umfang mit einer an den Körper des Patienten haftenden Substanz, wie Klebstoff, zu versehen. Als Klebstoff eignen sich beispielsweise Pektin-Cellulose-Verbindungen. Die abzudichtende Substanz soll allerdings ein einfaches Entfernen bzw. Abziehen des Absorptionskörpers vom Körper des Patienten ermöglichen. Ein solcher Absorptionskörper ist insbesondere zum Auflegen auf die Wunde und zur direkten Behandlung bei postoperativen Drainagesystemen geeignet.

Weitere Merkmale und Vorteile der Erfindung sind den nachstehenden Ausführungsbeispielen zu entnehmen. Die Ausführungsbeispiele sind anhand der Zeichnung näher erläutert, deren Figuren im einzelnen zeigen:
- Fig.1: einen flächenhaften Absorptionskörper in einer ersten Ausführungsform, in Draufsicht auf seine Flachseite;
- Figuren 2a bis 3: weitere Ausführungsformen des Absorptionskörpers, ebenso in Draufsicht auf seine Flachseite;
- Figuren 4 und 5: einen schematischen Schnitt durch den Absorptionskörper gemäß Figuren 1 bis 3, mit einer auf die Hülle aufgebrachten Klebeschicht und mit Schutzfolie bzw. mit Zugmittel;
- Figuren 6 und 7: einen schematischen Schnitt durch den Absorptionskörper mit jeweils zwei Lagen vom Absorptionsmaterial;
- Fig.8: einen schematischen Schnitt durch den Absorptionskörper mit einem an der Hülle angebrachten Folienabschnitt;
- Fig.9a: einen schematischen Schnitt durch den Absorptionskörper mit einer aus einem Folienabschnitt und einer perforierten Wand bestehenden Hülle;
- Fig.9b: einen schematischen Schnitt durch den Absorptionskörper gemäß Fig.9a, mit einer Abziehfolie;
- Fig.10a: eine innere Umhüllung zwischen dem Absorptionsmaterial und der Hülle, ebenso in einem schematischen Schnitt;
- Fig.10b: den Absorptionskörper mit einer zusätzlichen Zwischenlage;
- Figuren 11a bis 11c: den Absorptionskörper gemäß Figuren 1 bis 3, untergebracht in einem Auffangbeutel, in verschiedenen Ausführungen;
- Figuren 12a bis 12h: den in einem Auffangbeutel platzierten Absorptionskörper mit einem Zugmittel, jeweils vor und während der Entnahme des Absorptionsmaterials;
- Fig.13: den in einem grobmaschigen Netzbeutel platzierten Absorptionskörper gemäß Figuren 1 bis 3;
- Fig.14a: den Absorptionskörper gemäß Figuren 1 bis 3, untergebracht in einem wasserdichten Überzug;
- Fig.14b: den Absorptionskörper mit Überzug gemäß Fig.14a in Verwendung bei einem Urinableitsystem;
- Fig.15: ein Urinableitsystem gemäß Fig.14a, mit einem nachgeschalteten Urinbeutel;
- Figuren 16 und 17: den auf die Haut des Patienten aufgelegten Absorptionskörper gemäß Figuren 1 bis 3;
- Fig.18: den Absorptionskörper gemäß Fig.1 mit Befestigungsmittel;
- Figuren 19 und 20: den in einem Überzug angeordneten Absorptionskörper, aufgelegt auf die Wunde und dort befestigt;
- Fig.21: den Überzug gemäß Fig.14a mit einem Einschubfach, im Schnitt;
- Fig.22: einen Material-Abschnitt der Hülle, in einer perspektivischen Sicht,
- Fig.23: das Material der Hülle im Schnitt, in einer vergrößerten Darstellung,
- Fig.24: Detail einer Befestigung des Absorptionskörpers innerhalb des Auffangbeutels oder Überzuges,
und
- Figuren 25a und 25b: einen Ausstreifbeutel mit einer Trennwand, in einem schematischen Querschnitt.

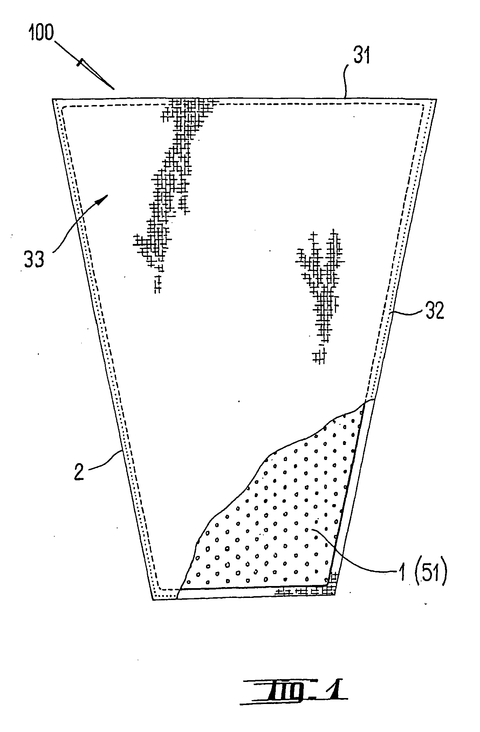

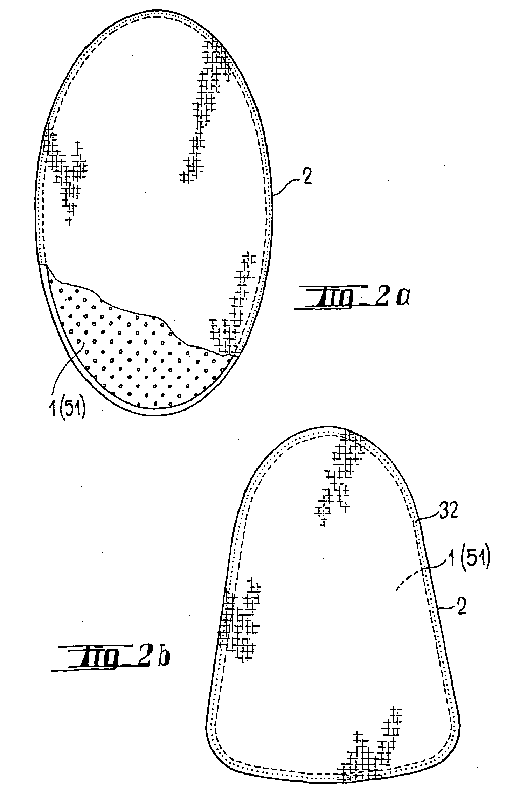

In Figuren 1 bis 2b ist ein Absorptionskörper in seiner ersten Ausführungsform (Bezugszahl 100) dargestellt. Die Varianten des Absorptionskörpers 100 unterscheiden sich voneinander lediglich durch ihre äußere Form. In Fig.1 ist ein trapezförmiger, in Fig.2a ein ovaler und in Fig.2b ein etwa tropfenförmiger Absorptionskörper gezeigt. Der Absorptionskörper 100 besteht aus einer Hülle 2 und einem innerhalb der Hülle 2 lose untergebrachten Absorptionsmaterial 1 in Form eines flachen Textil-Abschnittes 51.

Die Hülle 2 ist aus einem einstückigen, doppeltrapezförmigen Zuschnitt (nicht dargestellt) derart angefertigt, daß dieser um eine längs der Trapezbasis verlaufende Faltlinie 31 zusammengefaltet und an den übrigen Seitenkanten verschweißt (Naht 32) ist. Wie die Fig.1 zeigt, ist der Textil-Abschnitt 51 ebenfalls trapezförmig. Der trapezförmige Absorptionskörper 100 weist ein Basismaß von 11 cm und ein Maß an der Spitze von 5,5 cm mit einer Seitenlänge von 15 cm auf. Die Ausmaße können um einen Betrag von ± 2 cm variieren.

Die Hülle gemäß Figuren 2a und 2b besteht jeweils aus zwei gleichen, an ihrem Umfang miteinander verschweißten Seitenwänden.

Die Hülle 2 hat die Aufgabe, die Flüssigkeiten, wie Emulsionen und Lösungen, die atomare, elementare, proteinogene, hydrophile und langkettige Substanzen enthalten, in das Innere des Absorptionskörpers zu transportieren. Die Hülle 2 bildet zugleich eine Barriere für feste, grobe Ausscheidungen, so daß im wesentlichen nur die blut-, stuhl-, zell- oder enzymhaltigen oder infektiösen flüssigen Substanzen durch die Hülle hindurchtreten und vom Absorptionsmaterial 1 aufgenommen werden können.

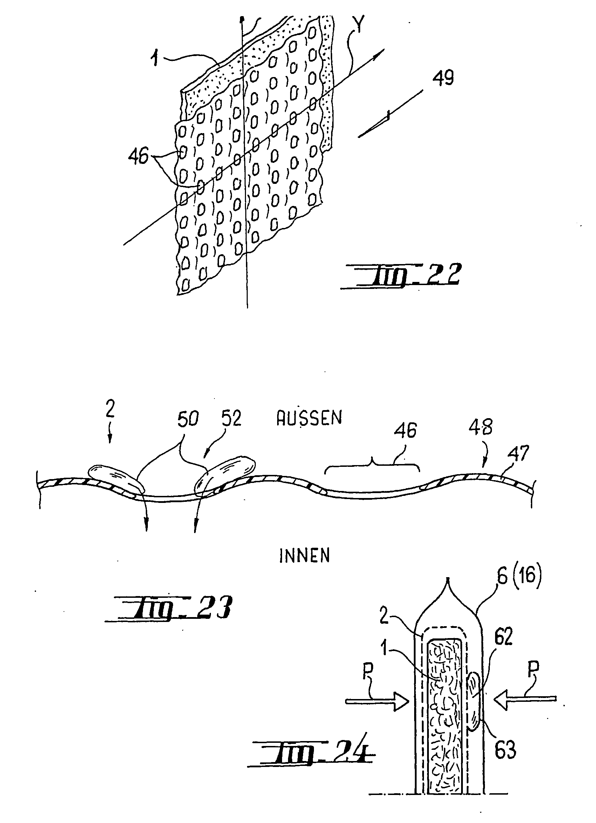

Die Figur 23 zeigt den Transportmechanismus von Flüssigkeiten. Die Hülle 2 besteht aus einer wasch- und desinfizierbaren, 30 µm dicken Polyesterfolie mit einer Vielzahl von in beiden X,Y-Richtungen (vgl. Material-Abschnitt 49; Fig.22) aufgereihten Maschen 46.

Wie den beiden Figuren 22, 23 zu entnehmen ist, ist das Material der Hülle 2 derart strukturiert, daß seine die Maschen 46 begrenzenden Fäden- bzw. Faserabschnitte 47 - im Schnitt durch die Hülle gesehen - jeweils etwa bogenförmig sind und mit ihren Bogen-Scheiteln 48 nach außen gerichtet sind. Mit anderen Worten sind die Maschen bzw. Perforationen dem im Inneren der Hülle 2 platzieren Absorptionsmaterial 1 (Textil-Material 51) zugewandt und liegen jeweils in einer quasi Mulde 52 einer sich in beiden X,Y-Richtungen erstrekkenden, etwa wellenförmigen Struktur.

Da das Material der Hülle 2 glatt und hydrophob ist, gleiten die Flüssigkeitsmoleküle 50 über die Außenfläche der Hülle und treten einfacher durch die Perforationen ins Innere der Hülle 2 hindurch, wo sie von dem Absorptionsmaterial aufgenommen werden können. Erfahrungsgemäß ist die Maschengröße zwischen 0,25 mm und 1,2 mm, im vorliegenden Fall etwa 0,5 mm optimal.

Selbstverständlich ist möglich, anstelle der beschriebenen eine andere Materialstruktur der Hülle zu wählen, beispielsweise eine wellenkammartige, bei der die Reihen von Perforationen jeweils in einer länglichen Mulde zwischen zwei Wellenkämmen liegen (nicht dargestellt).

Die mit einer ebenen Materialstruktur der Hülle durchgeführten Experimente haben gegenüber der welligen bzw. muldenartigen Struktur erheblich schlechtere Ergebnisse gebracht.

Der in Figuren 1 bis 2b gezeigte Absorptionskörper 100 ist als Basisprodukt gedacht, das sich mit verschiedenen zusätzlichen Elementen, die noch im weiteren beschrieben werden, ergänzen oder ausstatten läßt.

Der innere Textil-Abschnitt 51 besteht aus einem mehrlagigen, hochsaugfähigen Zellstoff-Vlies von einer Dicke etwa 2 mm. Die Außenschichten des Zellstoff-Vlieses sind ebenfalls perforiert, so daß die aufzusaugenden Flüssigkeitsmoleküle nicht nur aufgrund der Kapillarwirkung, sondern auch durch diese Teil-Perforationen in dessen Kern gelangen.

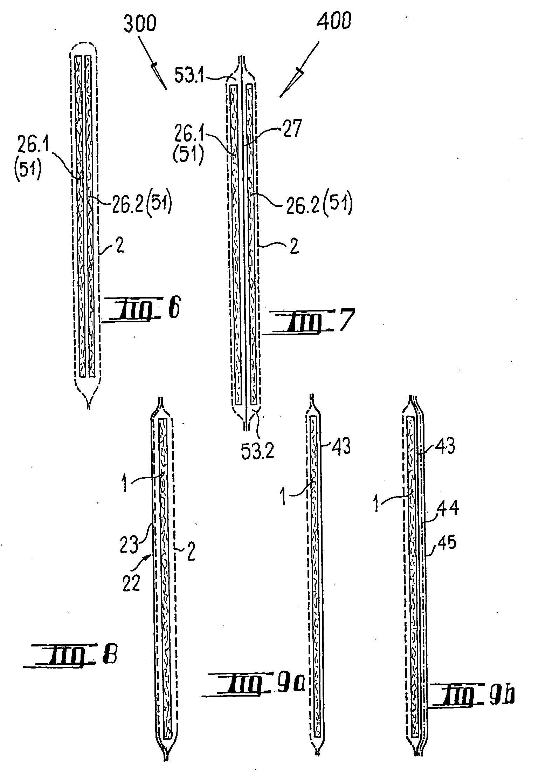

Die Figuren 6 und 7 zeigen weitere Ausführungsformen des Absorptionskörpers (Bezugszahlen 300 und 400). Als Absorptionsmaterial sind jeweils zwei Lagen 26.1, 26.2 des bereits beschriebenen Textil-Abschnittes 51 vorgesehen, wobei bei der Ausführung gemäß Fig.7 zwischen den Lagen 26.1, 26.2 eine flüssigkeitsundurchlässige Innenwand 27 angeordnet ist, die den Absorptionskörper 400 in zwei gleiche Kammern 53.1, 53.2 unterteilt. Anstelle der flüssigkeitsundurchlässigen Innenwand 27 kann eine flüssigkeits- und/oder gasdurchlässige Membran (nicht dargestellt) Verwendung finden.

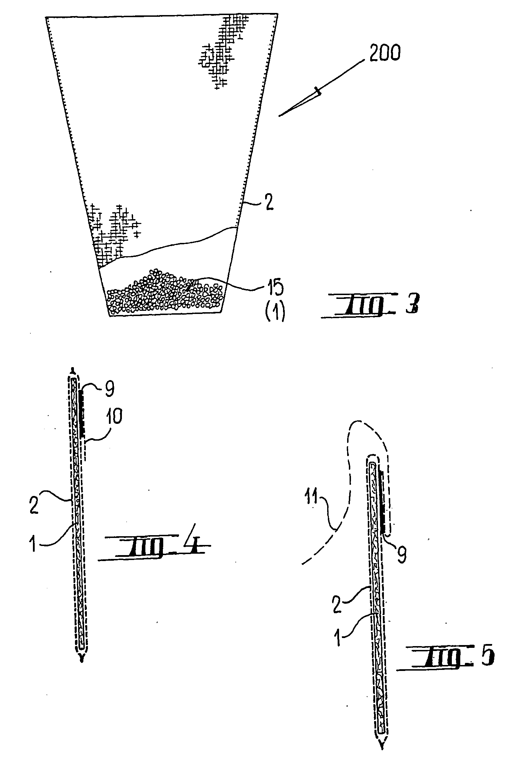

In Fig.3 ist ein trapezförmiger Absorptionskörper 200 dargestellt, bei dem anstelle eines inneren Textil-Abschnittes eine lose Schüttung 15 als Absorptionsmaterial vorgesehen ist. Die Schüttung besteht im vorliegenden Fall aus Natriumacrylat-Acrylsäure-Polymerisat in Granulatform.

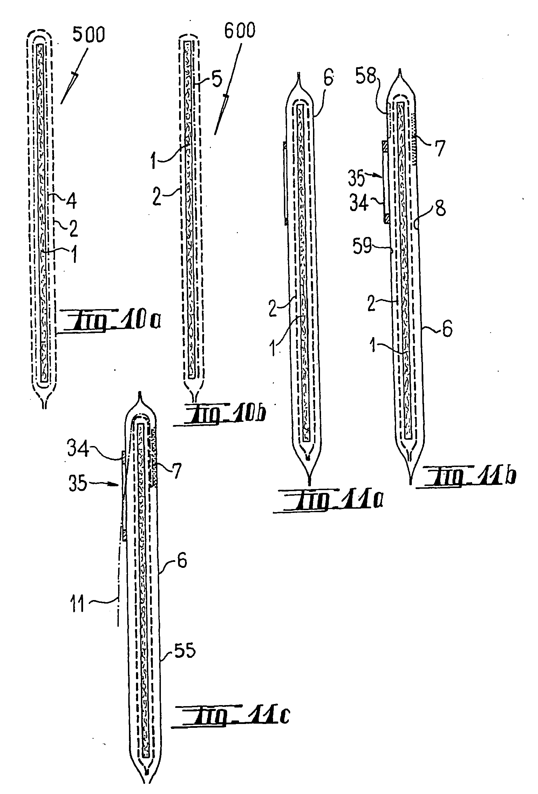

Bei einem in Fig. 10a gezeigten Absorptionskörper 500 ist zwischen der Hülle 2 und dem Absorptionsmaterial 1 eine weitere, innere, vollständige Umhüllung 4 aus Baumwolle angeordnet, deren Saugfähigkeit kleiner als die des eigentlichen, inneren Absorptionsmaterials (Textil-Abschnittes 51) ist. Die Umhüllung 4 erschwert den direkten Kontakt zwischen den Schleimhautzellen an der Wunde oder Körperhöhle und dem Absorptionsmaterial.

Bei einer in Fig.10b dargestellten, anderen Ausführungsform (Bezugszahl 600) ist anstelle der Umhüllung 4 eine Zwischenlage 5 in Form eines Materialabschnittes vorgesehen, die zwischen dem Absorptionsmaterial 1 und der Hülle 2 angeordnet ist. Die Zwischenlage 5 besteht ebenfalls aus Baumwolle und hat dieselbe Aufgabe, wie die Umhüllung 4.

Alle oben aufgeführten Absorptionskörper 100...600 können mit einer auf die Hülle 2 aufgebrachten Klebeschicht 9 versehen sein, wie der Fig.4 zu entnehmen ist. Die streifenförmige Klebeschicht 9 ist mit einer ebenso streifenförmigen, abziehbaren Schutzfolie 10 bedeckt. Um das Entfernen der Schutzfolie 10 zu erleichtern, endet diese mit einer Abziehlasche 54. Gemäß Fig.5 ist an der Klebeschicht 9 ebenfalls eine abziehbare Schutzfolie angebracht, die in gefalteter Form in ein Zugmittel 11 übergeht.

Die mit der Klebeschicht 9 und der abziehbaren Schutzfolie 10 bzw. mit dem Zugmittel 11 versehenen Absorptionskörper sind bestens für die in der Stoma-Versorgung verwendeten Auffangbeutel 6 geeignet. Zu diesem Zweck ist noch an einer Innenseite 8 des Auffangbeutels 6 ein Befestigungsbereich 7 vorgesehen, dessen Anordnung der Anordnung der Klebeschicht 9 an der Hülle 2 entspricht. Zur Applikation des Absorptionskörpers in den Auffangbeutel 6 soll nur die Klebeschicht 9 freigegeben werden, und zwar erst nach dem Einlegen des Absorptionskörpers. Eine solche Situation ist in Fig. 11c dargestellt. Der Befestigungsbereich 7 ist an der Innenseite 8 einer körperentfernten Wand 55 des Auffangbeutels 6 angeordnet, so daß das Zugmittel 11 um den Absorptionskörper gelegt und durch eine Öffnung 35 einer Befestigungsplatte 34 geführt ist. Diese Anordnung hat sich als besonders vorteilhaft erwiesen, weil die Lage des Absorptionskörpers sicher festgelegt ist. So kann der Absorptionskörper den mechanischen, von den festen Ausscheidungen stammenden Andrückkräften standhalten, so daß sein Quellvermögen voll ausgenutzt werden kann.

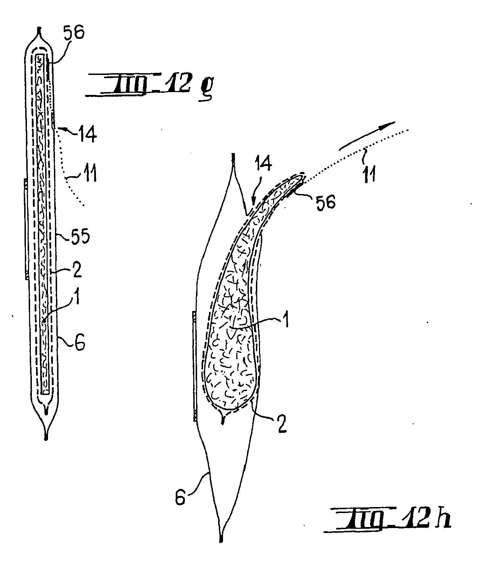

In Fig.12g ist eine andere Möglichkeit der Unterbringung des Absorptionskörpers im Inneren des Auffangbeutels 6 gezeigt. Das Zugmittel 11 ist durch einen Schlitz 14 geführt, der an der körperentfernten Wand 55 des Auffangbeutels 6 eingearbeitet ist. Das Zugmittel 11 ist bei 56 an der Hülle 2 befestigt, wobei im vorliegenden Fall keine Klebeschicht an der Hülle und kein Befestigungsbereich am Auffangbeutel 6 vorgesehen sind. Der aufgequollene, verbrauchte Absorptionskörper wird durch den Schlitz 14 aus dem Auffangbeutel 6 entfernt, wie die Fig.12h zeigt.

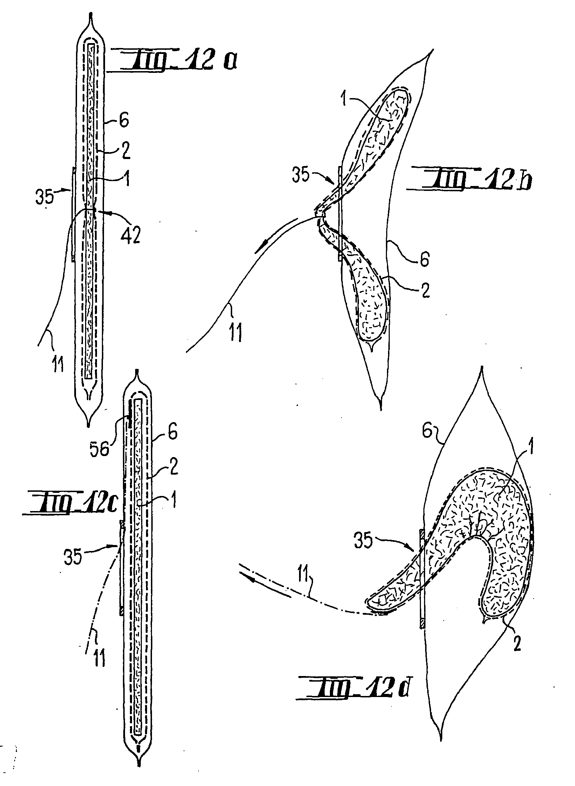

Wird der im Auffangbeutel 6 platzierte Absorptionskörper gegenüber der in Fig. 12g gezeigten Lage seitlich umgedreht, befindet sich der Befestigungspunkt 56 des Zugmittels 11 an der körpernahen Seite (vgl. Fig.12c), so daß das Zugmittel 11 direkt durch die Öffnung 35 in der Befestigungsplatte 34 geführt ist. Dies ermöglicht ein einfaches Herausziehen des Absorptionskörpers durch die Öffnung 35 (vgl. Fig.12d).

Die Figuren 12a und 12b zeigen eine ähnliche Konfiguration, bei der das Zugmittel 11 über eine in der Mitte des Absorptionskörpers angeordnete Naht 42 an dem Absorptionskörper befestigt ist.

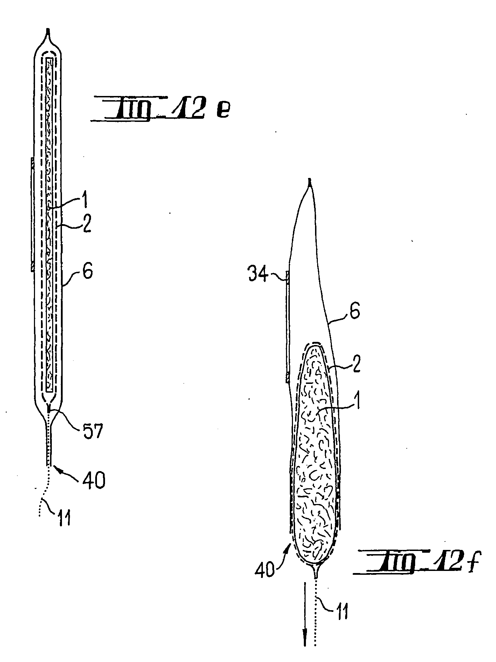

Bei einem an sich bekannten, eine untere Öffnung 40 aufweisenden Auffangbeutel 6 gemäß Figuren 12e und 12f wird der verbrauchte Absorptionskörper 100...600 ebenfalls mit Hilfe des Zugmittels 11 durch diese Öffnung 40 herausgezogen. Das Zugmittel 11 ist an einem unteren Rand 57 der Hülle 2 angebracht. Zur Verdeutlichung des Nutzungsprinzips ist der übliche Verschluß eines solchen Auffangbeutels, wie Klammer, weggelassen.

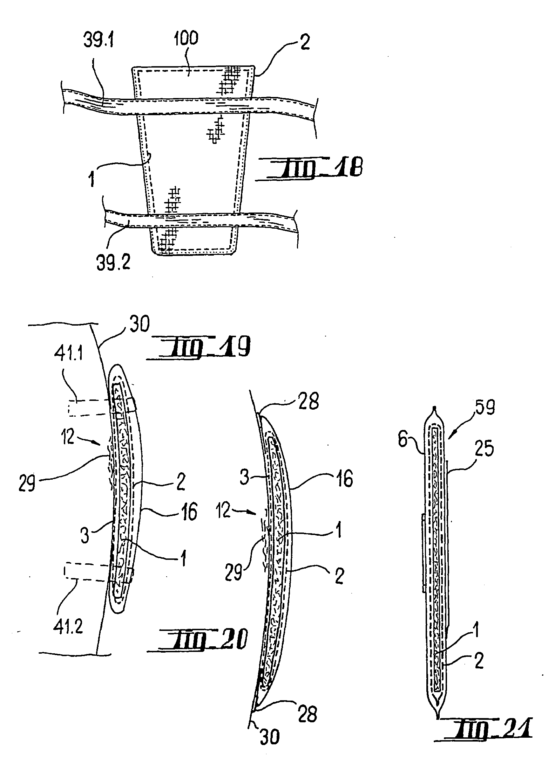

Gemäß Fig.11a ist der Absorptionskörper 100...600 lose im Auffangbeutel 6 untergebracht, wobei auf das Zugmittel und die Klebebefestigung verzichtet wurde. Bei einer ähnlichen Konfiguration (vgl. Fig.21) ist an einer körperentfernten Außenseite 59 des Auffangbeutels 6 ein Einschubfach 25 zur Unterbringung von Einmalhandschuhen vorgesehen.

Die Fig.11b stellt eine weitere Möglichkeit der Applikation des Absorptionskörpers in den Auffangbeutel 6 dar. Der Absorptionskörper ist entweder an der körperentfernten oder an der körpernahen Innenseite 8; 59 des Auffangbeutels 6 zu befestigen (Befestigungsbereiche 7; 58). Ein Zugmittel ist hier auch nicht vorhanden.

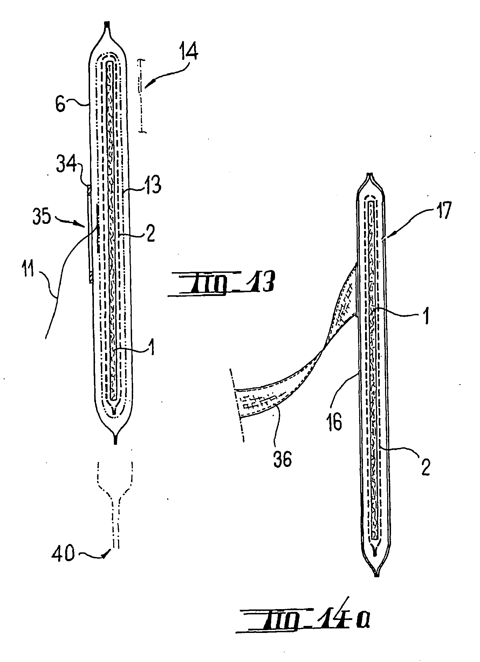

Den in einen grobmaschigen Netzbeutel 13 eingelegten Absorptionskörper 100....600 zeigt Fig.13. Der ebenfalls abgeflachte, aus Polyesterfäden hergestellte Netzbeutel 13 weist eine Maschengröße 5 mm auf. Seine Aufgabe ist, die räumliche Fixierung des Absorptionskörpers und somit der aufzusaugenden flüssigen Ausscheidungen zu verbessern. Der Netzbeutel 13 ist lose und entnehmbar im Auffangbeutel 6 untergebracht. Hierbei sind drei Entnahmemöglichkeiten mit Hilfe des Zugmittels 11 vorgesehen, und zwar durch die Öffnung 35 an der Befestigungsplatte 34, durch einen Schlitz 14 (wie bei Figuren 12g und 12h) oder durch eine untere Öffnung 40 (wie bei Figuren 12e und 12f).

Die Verwendungsbereiche des Absorptionskörpers gemäß Erfindung umfassen auch einen flächenhaften, flüssigkeitsundurchlässigen Überzug 16 (vgl. Fig.14a), welcher mit wenigstens einem Gurt 36 am Körper des Patienten zu befestigen ist. Der Überzug 16 ist mit einem an seiner körperentfernten Seite eingebrachten Schlitz 17 zur Entnahme oder zum Auswechseln des Absorptionskörpers versehen. Der Überzug 16 ist zur Verwendung bei Urinsystemen gedacht, allerdings ist nicht ausgeschlossen, diesen als Bereitschaftstasche für die Unterbringung von Ersatz-Absorptionskörper (-n) zu benutzen.

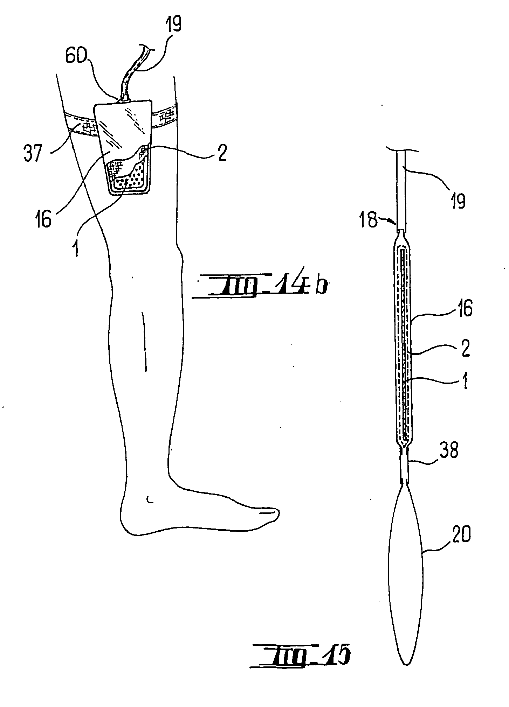

Ein am Oberschenkel des Patienten befestigter Überzug 16 ist in Fig. 14b gezeigt. Der Überzug 16 hängt an einem das Bein des Patienten umgebenden, leicht elastischen, textilen Gurt 37, der mit einem nicht dargestellten Klettverschluß zugemacht ist. Im Inneren des Überzuges 16 befindet sich ein Absorptionskörper, der in einem Teilschnitt angedeutet ist. An eine am oberen Rand des Überzuges 16 eingebrachte Öffnung 60 ist ein Blasenkatheter 19 angeschlossen. Das Urinsystem ist gemäß Fig.15 um einen Urinbeutel 20 erweitert, der über ein kurzes Verbindungsrohr 38 aus Kunststoff an den Überzug 16 angeschlossen ist.

Die weiteren Ausführungsformen (nicht dargestellt) sehen die Plazierung eines zweiten Überzuges zwischen dem ersten und dem Urinbeutel, bzw. das Anschließen des unteren, zweiten Überzuges (mit Absorptionskörper) anstelle des Urinbeutels 20 vor. So wird ein kaskadenartiges Urinableitsystem aufgebaut. Wird der obere Absorptionskörper bis zu seiner Kapazitätsgrenze aufgequollen, gelangt der Überschuß an Urin in den zweiten, unteren Absorptionskörper. Eine solche Lösung kann das Sicherheitsgefühl des Inkontinenz-Patienten verbessern.

Der den Absorptionskörper 100....600 umgebende Überzug 16 kann in Behandlung von Wunden verwendet werden. So zeigt die Fig.19 einen mittels zwei Klebebänder 41.1, 41.2 an der Haut 30 des Patienten Überzug 16, der jedoch eine körpernahe Öffnung 12 aufweist, damit die flüssigen, aus der Wunde 29 austretenden Substanzen direkt von dem innerhalb des Überzuges platzierten Absorptionskörper aufgesaugt werden können.

Wie die Fig.20 zeigt, ist am Umfang des Überzuges 16 ein den Wundbereich abdichtendes Klebestreifen 28 vorgesehen.

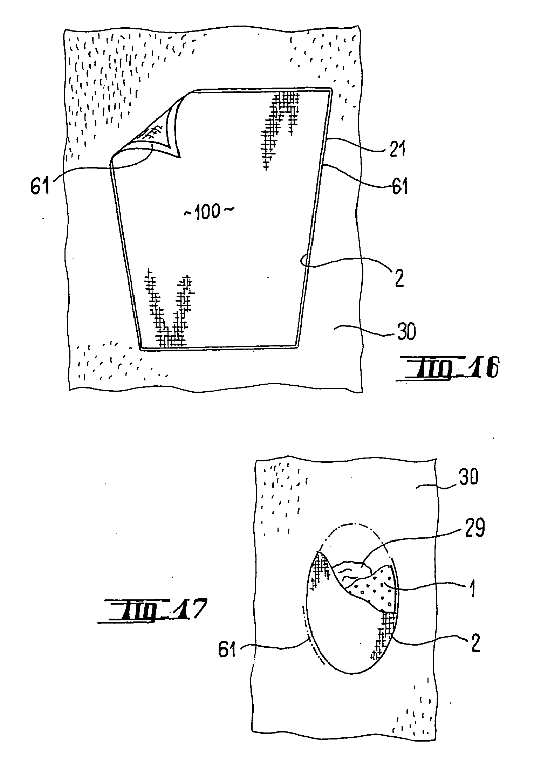

Der aus der Hülle 2 und dem Absorptionsmaterial 1 (insbesondere in Form des Textil-Abschnittes 51) bestehende Absorptionskörper 100....600 ist auch zur direkten Behandlung von Wunden geeignet, ohne einen zusätzlichen Überzug verwenden zu müssen. Dieser kann mit Klebestreifen oder Gurten 39.1, 39.2 (vgl. Fig.18), oder, wie die Figuren 16 und 17 zeigen, mit einem an seinem Umfang 21 abgebrachten Klebeband 61 abdichtend an der Haut 30 des Patienten befestigt sein. In Fig.16 ist ein trapezförmiger und in Fig.17 im Teilschnitt ein ovaler Absorptionskörper 100 gezeigt.

In den Figuren 8 bis 9b sind drei von dem Absorptionskörper 100 bis 600 abweichenden Ausführungsformen dargestellt. Gemäß Fig.8 ist an einer äußeren Flachseite 22 der Hülle 2 ganzflächig ein flüssigkeitsundurchlässiger Folienabschnitt 23 angebracht. Der Folienabschnitt 23 ist mit der Hülle 2 an ihrem Umfang verschweißt.

Wie die Fig.9a zeigt, ist eine Seitenwand der Hülle 2 durch einen flüssigkeitsundurchlässigen Folienabschnitt 43 ersetzt. Wird die Außenseite des Folienabschnittes 43 mit einer Klebeschicht oder Klebefolie 44 bedeckt, so ergibt sich eine in Fig.9b dargestellte Konfiguration. Die Klebefolie 44 ist noch mit einer Abziehfolie 45 versehen. Anstelle einer ganzflächig den Folienabschnitt 43 bedeckenden Klebe- und Abziehfolie können schmale Folienstreifen verwendet sein.

Die Fig.24 zeigt eine andere Möglichkeit der Fixierung des Absorptionskörpers im Auffangbeutel 6 oder im Überzug 16. Hierbei ist ein an der Hülle 2 angebrachtes kissenförmiges Befestigungselement 62 vorgesehen, das aus einem flüssigen Klebstoff und einem den Klebstoff umgebenden, sehr dünnen Film 63 besteht. Beim Andrücken z.B. mit dem Daumen und Zeigefinger in Richtung Pfeile P zerplatzt der Film 63, etwa wie bei "Knallerbsen", gibt den Klebstoff frei und bildet somit eine Klebeverbindung mit dem Auffangbeutel oder Überzug.

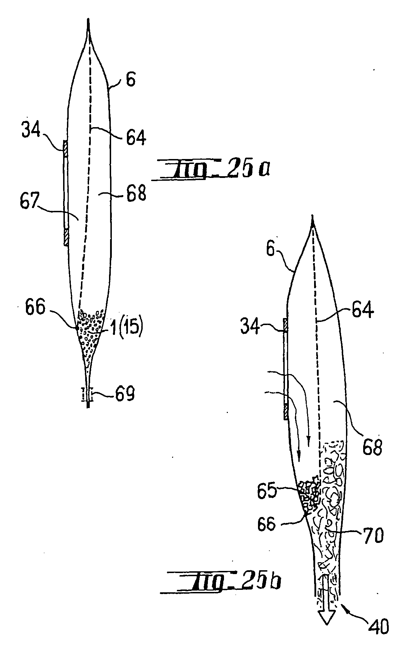

Schließlich ist den Figuren 25a und 25b eine besonders vorteilhafte Ausführungsform eines Auffangbeutels 6 mit unterer Öffnung 40 (sogenannter Ausstreifbeutel) zu entnehmen, bei der innerhalb des Auffangbeutels 6 anstelle der Hülle 2 eine Trennwand 64 angeordnet ist. Die Trennwand 64 besteht aus dem bereits bei den Figuren 22, 23 aufgeführten Material und hat auch dieselbe Aufgabe. Die Trennwand 64 reicht bis zu einer Schweißlinie 66 (hier sichtbar als Punkt) an der mit der Befestigungsplatte 34 versehenen Wand, und zwar unterhalb der Öffnung 35 und teilt das Innere des Auffangbeutels 6 in zwei Kammern 67, 68, von denen die körpernahe Kammer 67 nur für feste und die körperentfernte Kammer 68 nur für flüssige Ausscheidungen bestimmt ist. In der körperentfernten Kammer 68 befindet sich das Absorptionsmaterial 1 in Form der Schüttung 15. Die körpernahe Kammer 67 ist unten immer geschlossen, dagegen die körperentfernte Kammer 68 nach dem Entfernen einer Klammer 69, d.h. während des Ausstreifens (vgl. Fig.25b) offen ist.

Mit der Bezugszahl 70 sind die flüssigen Ausscheidungen bezeichnet. Die Menge an Absorptionsmaterial 1 (Schüttung 15) und dessen Saugfähigkeit sind so gewählt, daß die flüssigen Ausscheidungen 70 nicht durch die Öffnung 35 zurückfließen können.

Diese "Sortierung" der Ausscheidungen ist dadurch möglich, daß die bereits beschriebenen Bogen-Scheiteln 48 des Materials der Trennwand 64 der Befestigungsplatte 34 zugewandt sind.

Zur Erläuterung: Die Begriffe "untere", "obere" und dgl. beziehen sich auf den stehenden oder sitzenden Patienten, der einen Auffangbeutel bzw. einen Überzug mit darin untergebrachten Absorptionskörper trägt (benutzt). Die Begriffe "körpernahe" und "körperentfernte" beziehen sich auf den Körper des Patienten.

### Bezugszeichenliste

- 100, 200....: Absorptionskörper
- 1: Absorptionsmaterial
- 2: Hülle
- 4: Umhüllung
- 5: Zwischenlage
- 6: Auffangbeutel
- 7: Befestigungsbereich
- 8: Innenseite (v.6)
- 9: Klebeschicht
- 10: Schutzfolie
- 11: Zugmittel
- 12: Öffnung (v.16)
- 13: Netzbeutel
- 14: Schlitz (v.12)
- 15: Schüttung
- 16: Überzug
- 17: Öffnung (v.16)
- 18: Ende (v.19)
- 19: Blasenkatheter
- 20: Urinbeutel
- 21: Umfang
- 22: Flachseite
- 23: Folienabschnitt
- 25: Einschubfach
- 26.1, 26.2: Lage (v.1)
- 27: Innenwand
- 28: Klebestreifen
- 29: Wunde
- 30: Haut
- 31: Faltlinie
- 32: Naht
- 33: Oberfläche
- 34: Befestigungsplatte
- 35: Öffnung (v.34)
- 36: Gurt
- 37: Gurt
- 38: Verbindungsrohr
- 39.1, 39.2: Gurt
- 40: Öffnung
- 41.1, 41.2: Klebeband
- 42: Befestigungsnaht
- 43: (flüssigkeitsundurchlässige) Wand
- 44: Klebefolie
- 45: Abziehfolie
- 46: Masche
- 47: Faden- oder Faserabschnitt
- 48: Bogen-Scheitel
- 49: Material-Abschnitt
- 50: Flüssigkeitsmolekül
- 51: Textil-Abschnitt (v.1)
- 52: Mulde
- 53.1, 53.2: Kammer
- 54: Abziehlasche
- 55: Wand
- 56: Befestigung (v.11)
- 57: Rand (v.2)
- 58: Befestigungsbereich
- 59: Außenseite (v.6)
- 60: Öffnung (v.16)
- 61: Klebeband
- 62: Befestigungselement
- 63: Film
- 64: Trennwand
- 65: (feste) Auscheidungen
- 66: Schweißlinie
- 67: Kammer
- 68: Kammer
- 69: Klammer
- 70: (flüssige) Ausscheidungen

- P: Pfeil
- X,Y: Richtung (Fig.22)

## Patentansprüche

1. Absorptionskörper (100 ; 200 ;.......) zum Anschluss an Haut- und Schleimhautoberflächen, insbesondere zum Aufsaugen von flüssigen Ausscheidungen aus natürlichen und nicht-natürlichen, sowie aus erkrankungsbedingten menschlichen Körperöffnungen und -höhlen oder aus chirurgisch angelegten Organausleitungen, enthaltend eine Menge an Absorptionsmaterial (1), wie aufsaugendes Vlies oder ein Gemisch aus mehreren Absorptionssubstanzen, das mit wenigstens einer flüssigkeitsdurchlässigen Abdeckung hinterlegt ist, **dadurch gekennzeichnet, dass**
• das Absorptionsmaterial (1) von einer Hülle (2) umgeben ist, die aus einem uni- oder bidirektional flüssigkeitsdurchlässigen, schleimhautverträglichen bzw. haut verträglichen Natur- oder Kunststoff, wie perforierter Vlies oder maschiges Textilmaterial, besteht, an dem die Ausscheidungen nicht oder kaum haften und das die Flüssigkeiten durch sich durchtreten lässt und eine Barriere für feste grobe Ausscheidungen bildet und die Aufnahme bzw. das Aufsaugen von austretenden und/oder entfernungspflichtigen Substanzen durch das innerhalb der Hülle (2) angeordnete Absorptionsmaterial (1) ermöglicht,
• so dass der direkte Kontakt der Ausscheidungen mit der Haut bzw. Schleimhaut im angeschlossenen Zustand des Absorptionskörpers (100 ; 200 ;......) an die Körperöffnung oder -höhle eingeschränkt ist, und die äusfliessenden Ausscheidungen durch die Hülle (2) hindurchtreten, in das Absorptionsmaterial (1) gelangen und innerhalb der Hülle (2) räumlich fixiert verbleiben, und wobei
• das Material der Hülle eine perforierte Polyolefin-Folie aufweist, die derart strukturiert ist, dass sie Perforationen aufweist, die dem im Inneren der Hülle zugewandten Absorptionsmaterial zugewandt sind.

2. Absorptionskörper nach Anspruch 2 , **dadurch gekennzeichnet, dass** dieser flächenhaft ist.

3. Absorptionskörper nach einem der Ansprüche 1 bis 2, **dadurch gekennzeichnet, dass** das Absorptionsmaterial (1) im Wesentlichen aus Zellstoffasern gebildet ist.

4. Absorptionskörper nach einem der Ansprüche 1 bis 3, **dadurch gekennzeichnet, dass** das Absorptionsmaterial (1) mit einem Superabsorber-Granulat oder -Pulver durch setzt ist.

5. Absorptionskörper nach einem der vorhergehenden Ansprüche, **dadurch gekennzeichnet, dass** zwischen dem Absorptionsmaterial (1) und der äusseren Hülle (2) eine zusätzliche, innere Umhüllung (4) aus einem Material, wie Baum wolle oder Zellstoff, angeordnet ist, dessen Saugfähigkeit gering bzw. kleiner als die des eigentlichen Absorptionsmaterials (1) ist, wodurch der direkte Kontakt zwischen den Schleimhautzellen an der Wunde oder Körper höhle und dem Absorptionsmaterial erschwert ist.

6. Absorptionskörper nach einem der vorhergehenden Ansprüche, **dadurch gekennzeichnet, dass** zwischen dem Absorptionsmaterial und der äußeren Hülle (2) eine zusätzliche, innere Zwischenlage (5) in Form eines Materialabschnittes aus Baumwolle, Zellstoff oder dergleichen angeordnet ist, wodurch der direkte Kontakt zwischen den Schleimhautzellen an der Wunde oder Körperhöhle und dem Absorptionsmaterial erschwert ist.

7. Absorptionskörper nach einem der vorhergehenden Ansprüche, **dadurch gekennzeichnet, dass** an wenigstens einer Außenseite (22) der perforierten Hülle (2) ganzflächig ein flüssigkeitsundurchlässiger Folienabschnitt (23) angebracht ist.

8. Absorptionskörper nach einem der vorhergehenden. Ansprüche, **dadurch gekennzeichnet, dass** die Hülle (2) zwei flach liegende Lagen (26.1, 26.2) des Absorptionsmaterials (1) umgibt, die gleiche oder unterschiedliche Saugkraft aufweisen.

9. Absorptionskörper nach einem der vorhergehenden Ansprüche, **dadurch gekennzeichnet, dass** das zu absorbierende Material in Form einer Schüttung (15) in der Hülle (2) vorliegt.

10. Absorptionskörper nach einem der vorhergehenden Ansprüche, **dadurch gekennzeichnet, dass** dieser mit Befestigungsmitteln, wie Gurte, Klebeband, Klett- -oder Magnetverschluss, versehen ist, die es erlauben, den Absorptionskörper an beliebigen Stelle des Körpers des Patienten zu fixieren.

11. Absorptionskörper nach einem der vorhergehenden Ansprüche, **dadurch gekennzeichnet, dass** das Absorptionsmaterial (1) Eigenschaft hat, ein inneres, wundheilungsfördendes Feuchtklima zu erzeugen.

12. Absorptionskörper nach einem der vorhergehenden Ansprüche, **dadurch gekennzeichnet, dass** die Hülle (2) und/oder das Absorptionsmaterial (1) - in Draufsicht auf ihre/seine Flachseite gesehen - an ihrem/seinem Umfang (21) mit einer an den Körper des Patienten haftenden Substanz, wie Klebstoff, beschichtet oder getränkt ist.

13. Absorptionskörper nach einem der vorhergehenden Ansprüche, **dadurch gekennzeichnet, dass** dieser ein auf die Wunde oder Körperhöhle auflegbarer Flüssigkeitsspeicher ist.

14. Verwendung eines Absorptionskörpers nach wenigstens einem der Ansprüche 1 bis 13 zum direkten Aufsaugen von flüssigen Ausscheidungen aus natürlichen oder erkrankungsbedingten Körperöffnungen und -höhlen, wie tiefen Wunden oder offenen Abszessen.

15. Verwendung eines Absorptionskörpers nach wenigstens einem der Ansprüche 1 bis 13 zur direkten Behandlung bei postoperativen Drainagesystemen.
